(19) Europäisches Patentamt / European Patent Office / Office européen des brevets

(11) **EP 4 117 508 B1**

(12) **FASCICULE DE BREVET EUROPEEN**

(45) Date de publication et mention de la délivrance du brevet:
**12.03.2025 Bulletin 2025/11**

(21) Numéro de dépôt: **21711234.1**

(22) Date de dépôt: **12.03.2021**

(51) Classification Internationale des Brevets (IPC):
**G06F 3/01** *(2006.01)*  **A61B 5/00** *(2006.01)*
**A61B 5/11** *(2006.01)*  **A61B 5/103** *(2006.01)*
**A61F 2/72** *(2006.01)*  **A61B 5/389** *(2021.01)*

(52) Classification Coopérative des Brevets (CPC):
**G06F 3/015; A61B 5/389; A61B 5/6824;**
**A61B 5/744; A61B 5/7455; G06F 3/011;**
**G06F 3/016;** A61B 5/1107; A61B 5/296;
A61B 2560/0223; A61F 2/72

(86) Numéro de dépôt international:
**PCT/EP2021/056373**

(87) Numéro de publication internationale:
**WO 2021/180945 (16.09.2021 Gazette 2021/37)**

(54) **MÉTHODE DE CONTRÔLE D'UN MEMBRE D'UN AVATAR VIRTUEL PAR LES ACTIVITÉS MYOÉLECTRIQUES D'UN MEMBRE D'UN SUJET ET SYSTÈME ASSOCIÉ**

VERFAHREN ZUR STEUERUNG EINER EXTREMITÄT EINES VIRTUELLEN AVATARS MITTELS MYOELEKTRISCHER AKTIVITÄTEN EINER EXTREMITÄT EINER PERSON UND SYSTEM DAFÜR

METHOD FOR CONTROLLING A LIMB OF A VIRTUAL AVATAR BY MEANS OF THE MYOELECTRIC ACTIVITIES OF A LIMB OF AN INDIVIDUAL AND SYSTEM THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.03.2020 FR 2002472**

(43) Date de publication de la demande:
**18.01.2023 Bulletin 2023/03**

(73) Titulaires:
• **Université de Bordeaux**
  **33000 Bordeaux (FR)**
• **Centre national de la recherche scientifique**
  **75016 Paris (FR)**

(72) Inventeurs:
• **HALGAND, Christophe**
  **33240 SAINT ANDRÉ DE CUBZAC (FR)**
• **GUEMANN, Matthieu**
  **33200 BORDEAUX (FR)**
• **GOULLET DE RUGY, Aymar**
  **33200 BORDEAUX (FR)**
• **CATTAERT, Daniel**
  **33470 GUJAN-MESTRAS (FR)**

(74) Mandataire: **Icosa**
  **83 avenue Denfert-Rochereau**
  **75014 Paris (FR)**

(56) Documents cités:
**US-A1- 2014 198 034**

• **AARON TABOR ET AL: "Designing Game-Based Myoelectric Prosthesis Training",** PROCEEDINGS OF THE 2017 CHI CONFERENCE ON HUMAN FACTORS IN COMPUTING SYSTEMS , CHI '17, ACM PRESS, NEW YORK, NEW YORK, USA, 2 May 2017 (2017-05-02), pages 1352 - 1363, XP058337746, ISBN: 978-1-4503-4655-9, DOI: 10.1145/ 3025453.3025676

- GAURAVKUMAR K PATEL ET AL: "Context-dependent adaptation improves robustness of myoelectric control for upper-limb prostheses", JOURNAL OF NEURAL ENGINEERING, INSTITUTE OF PHYSICS PUBLISHING, BRISTOL, GB, vol. 14, no. 5, 20 September 2017 (2017-09-20), pages 56016, XP020320132, ISSN: 1741-2552, [retrieved on 20170920], DOI: 10.1088/1741-2552/AA7E82

**Description**

La présente invention concerne une méthode de contrôle d'un membre d'un avatar virtuel par les activités myoélectriques d'un membre d'un sujet.

La présente invention vise en particulier la population des personnes victimes d'amputations et notamment des amputations du membre supérieur.

Certaines personnes amputées ont une prothèse myoélectrique, c'est-à-dire une prothèse de contact dont les mouvements sont commandés à l'aide de signaux électriques provenant des muscles de l'utilisateur.

Une des principales problématiques rencontrées par les patients porteurs de prothèses myoélectriques est la charge cognitive associée à la commande et l'asservissement visuel permanent que nécessite son utilisation.

Le contrôle myoélectrique nécessaire à la commande des prothèses est basé sur l'activation de deux muscles antagonistes et sur la qualité de la dissociation du contrôle de ces muscles. Un tel contrôle est long et nécessite beaucoup d'entraînement.

L'action est rendue d'autant plus difficile que les patients n'ont aucune information sur l'état de la prothèse autre que celui offert par le retour visuel. Ils doivent rester concentrés sur un objet à atteindre et surveiller l'action que la prothèse est en train de faire. Cela est extrêmement coûteux en termes de ressource cognitives, telles que la concentration et la commande musculaire. A cause de cela, de nombreux patients abandonnent leurs prothèses qui deviennent ce qu'on appelle communément les « prothèses placards ».

Pour ces différentes raisons, les prothèses sont aujourd'hui sous-utilisées par rapport à leurs capacités.

L'article de AARON TABOR et al: "Designing Game-Based Myoelectric Prosthesis Training" in Design and Cognitive Impairment, 2017, concerne un jeu développé pour entraîner des personnes amputées à utiliser leur prothèse myoélectrique. Ce document expose le développement d'une interface visuelle ("The Falling of Momo"). Le personnage du jeu est contrôlé grâce à un bracelet à électrodes placé sur le membre amputé, selon un contrôle proportionnel identique à celui de la commande des prothèses myoélectriques. L'intensité de la contraction musculaire perçue par les électrodes est proportionnelle au déplacement du personnage de jeu, offrant un retour visuel à l'utilisateur.

US 2014/198034 divulgue un bracelet à électrodes pour son utilisation avec des lunettes à écran intégré fonctionnant par la reconnaissance de gestes. Les contractions musculaires perçues par les électrodes lors de gestes prédéterminés, par exemple tendre l'index et fléchir ou étendre le poignet, permettent de réaliser des actions sur l'écran des lunettes, par exemple faire défiler un menu déroulant. La reconnaissance de geste peut être confirmée par un retour haptique, en appliquant un signal discret en tout ou rien en fin de geste).

L'article de GAURAVKUMAR K PATEL et al: "Context-dependent adaptation improves robustness of myoelectric control for upper-limb prostheses" in Journal of Neural Engineering vol.14, no.5 (2017), décrit un protocole pour améliorer le contrôle d'une prothèse myoélectrique d'avant-bras à l'aide d'un bracelet à électrodes et selon un modèle d'apprentissage. Les signaux obtenus par les électrodes sont traités pour en obtenir l'enveloppe. Les enveloppes des signaux d'activité musculaire sont utilisées dans un algorithme de reconnaissance de geste. Le signal de sortie de cet algorithme est une estimation du niveau d'activation normalisée associée à une fonction de la prothèse, par exemple l'ouverture de la main ou la rotation du poignet. Sur la base de ce signal de sortie sont calculés un seuil d'activité et un gain afin de définir les informations de contrôle-commande envoyées à la prothèse pour la mettre en mouvement. Le seuil d'activité est modifié afin d'éviter des activités parasites des autres fonctions. Le gain est fixé par une fonction.

Un but de la présente invention est de proposer une méthode permettant d'améliorer l'apprentissage de la dissociation musculaire et le travail fin des contractions musculaires.

A cet effet, la présente invention a pour objet une méthode de contrôle d'un membre d'un avatar virtuel par les activités myoélectriques d'un membre d'un sujet telle que définie dans la revendication 1.

Des aspects avantageux de l'invention sont définis dans les revendications dépendantes 2 à 5.

L'invention a également pour objet un système adapté pour mettre en œuvre la méthode de contrôle d'un membre d'un avatar virtuel par les activités myoélectriques d'un membre d'un sujet tel que défini dans la revendication 6.

Des aspects avantageux de l'invention sont définis dans les revendications dépendantes 7 à 11.

Ces caractéristiques et avantages de l'invention apparaîtront à la lecture de la description qui va suivre, donnée uniquement à titre d'exemple non limitatif, et faite en référence aux dessins annexés, sur lesquels :

- la figure 1 est une vue schématique d'un système selon un mode de réalisation de l'invention ;
- la figure 2 est une vue schématique d'un sujet portant une prothèse myoélectrique ; et
- la figure 3 est un graphique représentant la distribution des écarts à la cible pour chaque mouvement au cours d'essais de mouvements d'allers-retours entre deux cibles à l'aide d'un avatar virtuel pour trois types de retours sensoriels et une configuration en l'absence de tout retour sensoriel chez des sujets non-amputés.

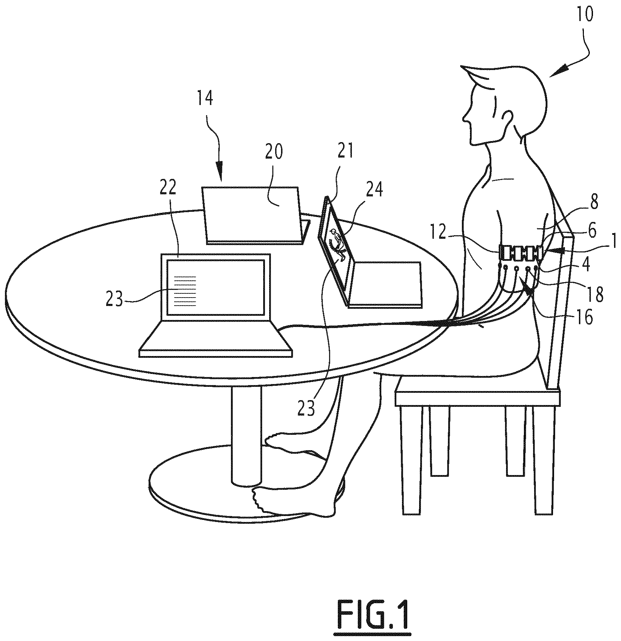

Sur la figure 1, un système 1 adapté pour mettre en oeuvre la méthode selon l'invention est représenté.

Le système 1 comprend un dispositif de mesure 4 de signaux d'activité myoélectrique. Les signaux d'activité myoélectrique proviennent des influx électriques transmis par des fibres nerveuses à un muscle et engendrant la contraction de ce muscle.

Le dispositif de mesure 4 comprend une pluralité d'électrodes 6. Les électrodes 6 sont par exemple des électrodes tri-phasiques de surface adaptées pour être placées sur un membre 8 d'un sujet 10 et recevoir les signaux d'activité myoélectrique issus des muscles du membre 8 de ce sujet 10.

Les électrodes 6 sont typiquement configurées pour acquérir des signaux d'activité myoélectrique.

En particulier, les électrodes 6 sont configurées pour acquérir un premier et un deuxième signal d'activité myoélectrique brut de calibration résultant de contractions d'intensités variables d'un premier et d'un deuxième muscles antagonistes, par exemple le biceps et le triceps.

Les électrodes 6 sont typiquement en outre configurées pour acquérir un premier et un deuxième signal d'activité myoélectrique brut résultant de contractions du premier et du deuxième muscles antagonistes du membre 8 du sujet 10, pendant une durée déterminée.

En particulier, le dispositif de mesure 4 de signaux d'activité myoélectrique comporte huit électrodes 6.

La fréquence d'acquisition des électrodes 6 est par exemple de 200 Hz. Une telle fréquence est particulièrement adaptée pour obtenir l'enveloppe du signal.

Cependant, d'autres valeurs de fréquences sont envisageables, par exemple comprises entre 100 Hz et 2000 Hz afin d'obtenir des signaux plus précis.

Le dispositif de mesure 4 se présente typiquement sous la forme d'un bracelet flexible. Une telle forme permet de placer le dispositif de mesure 4 autour d'un membre 8, le membre 8 étant par exemple un bras, la partie résiduelle d'un avant-bras ou moignon, ou d'un bras. Les électrodes 6 sont alors disposées en cercle et vers l'intérieur du cercle.

Les électrodes 6 sont reliées entre elles par tout matériau adapté pour former un bracelet. Le bracelet est par exemple extensible. Selon une variante, le bracelet se décline suivant plusieurs tailles adaptées à des morphologies différentes et pour s'adapter au membre supérieur comme au membre inférieur. Le bracelet peut comprendre en outre un dispositif de fermeture, par exemple un système auto-agrippant, une boucle, des boutons pression ou toute autre option envisageable.

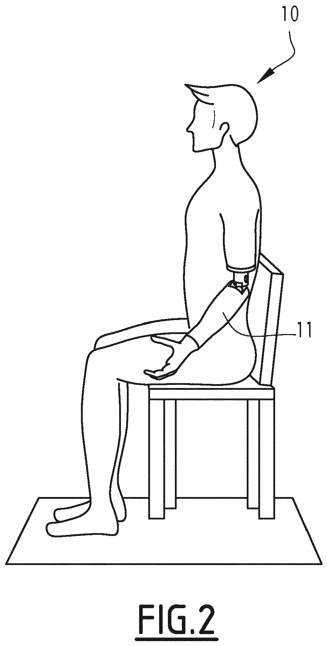

D'autres formes du dispositif de mesure 4 sont envisageables, par exemple les électrodes ne sont pas reliées entre elles, ou le dispositif de mesure 4 est intégré à une prothèse myoélectrique 11 telle que visible sur la figure 2. Par prothèse myoélectrique, il est entendu une prothèse de contact fonctionnant à l'aide de signaux d'activité myoélectrique et commandée par son utilisateur.

Le système 1 comprend également un dispositif de communication à distance 12.

Le dispositif de communication à distance 12 est typiquement un dispositif de communication radio.

Par exemple, le dispositif de communication à distance 12 est un dispositif de communication Bluetooth ou wifi.

Le dispositif de communication à distance 12 est configuré pour transmettre les activités myoélectriques relevées par les électrodes 6 à un dispositif de traitement 14 des activités myoélectriques.

Selon un mode de réalisation avantageux, le système 1 comprend également un dispositif de retour sensoriel 16.

Le dispositif de retour sensoriel 16 comprend par exemple une pluralité d'éléments vibrants 18.

Le dispositif de retour sensoriel 16 se présente typiquement sous forme de bracelet. Par exemple, le dispositif de retour sensoriel 16 se présente sous forme de bracelet élastique. De préférence, le dispositif de retour sensoriel 16 et le dispositif de mesure 4 sont solidaires.

Par exemple, le dispositif de retour sensoriel 16 comprend seize éléments vibrants 18. En variante, le dispositif de retour sensoriel 16 comprend un nombre différent d'éléments vibrants 18, par exemple six éléments vibrants.

Le dispositif de retour sensoriel 16 permet d'offrir un retour sensoriel au sujet 10 suite à une contraction musculaire entraînant la détection d'une activité myoélectrique par les électrodes 6. Son fonctionnement sera explicité plus loin.

Le dispositif de traitement 14 comprend au moins un processeur. Le dispositif de traitement 14 comprend en outre une mémoire propre à enregistrer les activités myoélectriques relevées.

Le dispositif de traitement 14 représenté sur la figure 1 comprend trois ordinateurs 20, 21, 22.

De préférence, le dispositif de traitement 14 comprend au moins un dispositif d'affichage 23.

Sur la figure 1, chaque ordinateur 20, 21, 22 comprend un dispositif d'affichage 23. Le dispositif d'affichage 23 est typiquement un écran.

Les trois ordinateurs 20, 21, 22 sont configurés pour communiquer entre eux, par exemple à l'aide d'un switch paramétré pour interagir en réseau local.

Le dispositif de mesure 4 de signaux d'activité myoélectrique est configuré pour communiquer à distance avec le dispositif de traitement 14. Dans l'exemple de la figure 1, le dispositif de mesure 4 est configuré pour communiquer à distance avec un premier ordinateur 20.

Le premier ordinateur 20 maître est configuré pour recevoir les signaux d'activité myoélectrique bruts acquis par les électrodes 6 et transmis par le dispositif de communication à distance 12, traiter lesdits signaux d'activité myoélectrique bruts et transmettre les signaux d'activité myoélectrique traités, en particulier à un deuxième ordinateur 21 et à un troisième ordinateur 22.

Avantageusement, le premier ordinateur 20 est configuré pour identifier deux électrodes 6 du dispositif de mesure 4 permettant une acquisition maximale des signaux d'activité myoélectrique de chacun des deux muscles antagonistes.

Le premier ordinateur 20 est configuré pour déterminer des activités myoélectriques minimales et maximales pour chacun des deux muscles antagonistes à partir d'un signal d'activité myoélectrique brut de calibration acquis.

A cette fin, le premier ordinateur 20 est également adapté pour mettre à valeur moyenne nulle le signal d'activité myoélectrique brut de calibration acquis par les électrodes 6.

Le premier ordinateur 20 est en outre configuré pour déterminer la valeur absolue du signal d'activité myoélectrique brut de calibration mis à valeur moyenne nulle.

Le premier ordinateur 20 est configuré pour filtrer le signal en valeur absolue pour en obtenir l'enveloppe.

Par exemple, le premier ordinateur 20 comprend un filtre Butterworth de type passe-bas d'ordre 2 et de fréquence de coupure 1,5 Hz configuré pour filtrer le signal obtenu pour en obtenir l'enveloppe.

Le premier ordinateur 20 est adapté pour déterminer un seuil d'activité myoélectrique, appelé offset EMGx, en-dessous duquel la contraction du muscle n'est pas obligatoirement volontaire et ne doit pas être prise en considération afin d'éviter de perturber un mouvement produit.

Le premier ordinateur 20 est configuré pour ne prendre en compte que les valeurs relevées entre le seuil déterminé et la valeur maximale de l'enveloppe du signal filtré.

Le premier ordinateur 20 est configuré pour normaliser les valeurs de l'enveloppe du signal filtré.

Le premier ordinateur 20 est typiquement configuré pour attribuer la valeur 0 au seuil d'activité myoélectrique précédemment calculé, et pour attribuer la valeur 1 à la valeur maximale de l'enveloppe du signal filtré.

En particulier, le premier ordinateur 20 est configuré pour déterminer un coefficient de conversion de l'activité myoélectrique normalisée en une composante de vitesse de déplacement d'un membre d'un avatar virtuel 24.

Par exemple, le coefficient de conversion est déterminé de la façon suivante pour le déplacement du bras de l'avatar. Classiquement, la rotation très rapide du coude est de 4 rad/s. Cette valeur est prise pour point de départ, et le sujet 10 donne son ressenti vis-à-vis de cette vitesse. Le coefficient de conversion est augmenté ou diminué afin que le ressenti de vitesse soit en accord avec l'activité musculaire du sujet 10. Ce coefficient est propre à chaque muscle.

Le premier ordinateur 20 est typiquement configuré pour attribuer, pour chaque muscle, une composante de vitesse nulle lorsque la valeur normalisée attribuée à la valeur de l'enveloppe du signal filtré est 0, et pour attribuer une composante de vitesse maximale lorsque la valeur attribuée à la valeur normalisée de l'enveloppe du signal filtré est 1.

En particulier, le premier ordinateur 20 est propre à attribuer, pour chaque muscle, à chaque valeur normalisée de l'enveloppe du signal filtré, une composante de vitesse comprise entre la composante de vitesse nulle et la composante de vitesse maximale.

Le premier ordinateur 20 est en outre configuré pour convertir l'activité myoélectrique normalisée en une composante de vitesse de déplacement du membre de l'avatar virtuel 24 par application du coefficient de conversion.

Le premier ordinateur 20 est configuré pour déterminer une vitesse et une direction de déplacement du membre de l'avatar virtuel 24 affiché par le deuxième ordinateur 21 par soustraction des composantes de vitesse obtenues pour chacun des muscles à un instant donné. Par exemple, dans le cas de la rotation d'un coude, la soustraction des valeurs de vitesse permet de déterminer une vitesse de flexion ou d'extension d'un coude d'un avatar virtuel 24 affiché par le dispositif d'affichage 23 du deuxième ordinateur 21.

Le dispositif d'affichage 23 du deuxième ordinateur 21 est configuré pour afficher l'avatar virtuel 24 représentant au moins un membre correspondant au mouvement qui doit être commandé par les activités myoélectriques. Il peut s'agir du bras ou d'une jambe, d'un corps entier ou un d'hémicorps en fonction de l'application choisie.

L'arrêt des contractions musculaires entraîne par exemple l'arrêt du mouvement, le positionnement du coude de l'avatar virtuel 24 est alors en position fixe qui correspond à la dernière position atteinte.

Selon des variantes, la vitesse calculée est une vitesse d'ouverture ou de fermeture de main d'un avatar virtuel, ou une vitesse de rotation de poignet d'un avatar virtuel, ou une vitesse de flexion/extension de genou ou de la cheville d'un avatar virtuel, ou toute autre option envisageable.

Le premier ordinateur 20 est configuré pour transmettre une commande de mouvement au deuxième ordinateur 21.

La commande de mouvement comprend typiquement la vitesse et la direction de déplacement déterminées.

De préférence, le premier ordinateur 20 est configuré pour déterminer un paramètre sensoriel du membre de l'avatar virtuel 24 à associer à au moins un élément vibrant 18 et à retranscrire en vibrations.

Le paramètre sensoriel est par exemple choisi parmi un angle de flexion d'un coude de l'avatar virtuel 24, un angle d'élévation antérieure d'une épaule de l'avatar virtuel 24, un angle d'abduction/adduction de l'épaule de l'avatar virtuel 24, un angle de rotation médiale ou latérale de l'épaule de l'avatar virtuel 24, un angle de flexion d'un poignet de l'avatar virtuel 24, un angle de rotation radial ou ulnaire de l'avatar virtuel 24, un degré d'ouverture d'une main de l'avatar virtuel 24, un degré d'ouverture d'une prise en pince digitale impliquant le pouce et un ou plusieurs autres doigts de la main de l'avatar virtuel 24, une force de serrage d'une main de l'avatar virtuel 24, un angle de flexion d'un genou de l'avatar virtuel 24, un angle de flexion de la cheville de l'avatar virtuel 24, une vitesse de rotation de l'un des mouvements mentionnés dans la présente liste, ou toute autre modalité sensorielle susceptible d'être substituée par des vibrations, telle que la chaleur ou la configuration d'une prothèse myoélectrique 11 du sujet 10.

La configuration de la prothèse myoélectrique 11 correspond au choix d'un des modes de la prothèse myoélectrique 11 en cours d'utilisation pour savoir sur quelle articulation l'activation musculaire va jouer. Par exemple si la prothèse est dans un mode appelé « prono-supination du poignet », les vibrations signaleront le mouvement de prono-supination du poignet. De même, dans un mode appelé « coude », les vibrations signaleront les mouvements du coude.

Le deuxième ordinateur 21 est dédié à l'interface visuelle avec le sujet 10.

Selon une variante non représentée, le deuxième ordinateur est directement intégré au premier ordinateur 20. Cela permet de réduire le nombre d'interfaces.

Le deuxième ordinateur 21 est configuré pour recevoir la commande de mouvement transmise par le premier ordinateur 20.

Dans un mode de réalisation avantageux, le troisième ordinateur 22 est configuré pour activer le dispositif de retour sensoriel 16.

Selon une variante non représentée, le troisième ordinateur est directement intégré dans le bracelet d'électrodes 6 au moyen d'un microcontrôleur et à un système de commande par modulation de largeur d'impulsion.

Le premier ordinateur 20 est configuré pour envoyer au troisième ordinateur 22 les activités myoélectriques traitées et converties en une valeur instantanée du paramètre sensoriel par application du coefficient de conversion.

Le troisième ordinateur 22 est typiquement configuré pour déterminer des fourchettes de valeurs du paramètre sensoriel à associer à au moins un élément vibrant 18.

Le troisième ordinateur 22 est typiquement configuré pour activer au moins un élément 18 correspondant à une fourchette de valeurs du paramètre sensoriel préalablement déterminée.

Le troisième ordinateur 22 comprend par exemple un programme de lecture configuré pour faire la correspondance entre la valeur instantanée du paramètre sensoriel et l'activation de l'élément vibrant 18 correspondant.

Le troisième ordinateur 22 est alors configuré pour activer l'élément vibrant 18 correspondant.

Le dispositif de retour sensoriel 16 est connecté au troisième ordinateur 22, par exemple à l'aide de câbles.

Dans la variante où le troisième ordinateur 22 est directement intégré au bracelet d'électrodes 6, les électrodes 6, le dispositif de retour sensoriel 16 et le troisième ordinateur 22 sont regroupés.

De préférence, le troisième ordinateur 22 est configuré pour activer les éléments vibrants 18 de manière sélective et discrète.

Par exemple, le troisième ordinateur 22 est propre à déterminer le nombre et la position des éléments vibrants 18 à activer en fonction de fourchettes de valeurs d'un paramètre sensoriel choisi.

De préférence, dans chaque fourchette de valeurs du paramètre sensoriel, un seul élément vibrant 18 déterminé est activé.

De préférence encore, à une fourchette de valeurs du paramètre sensoriel est attribué un unique élément vibrant 18 distinct pour chaque fourchette de valeurs.

Selon l'exemple où le paramètre sensoriel est l'angle de flexion du coude, lorsque le sujet 10 contracte le biceps et/ou le triceps, les électrodes 6 sont configurées pour acquérir un premier et un deuxième signal d'activité myoélectrique brut résultant de contractions du biceps et du triceps, respectivement ; pendant une durée déterminée, le dispositif de communication à distance 12 est configuré pour transmettre le premier et le deuxième signal d'activité myoélectrique brut au premier ordinateur 20, le premier ordinateur 20 est configuré pour traiter le premier et le deuxième signal d'activité myoélectrique brut et pour transmettre au deuxième ordinateur 21 une commande de déplacement du bras de l'avatar virtuel 24 et au troisième ordinateur 22 une position instantanée du bras de l'avatar virtuel 24, le deuxième ordinateur 21 est configuré pour afficher le déplacement du bras de l'avatar virtuel 24 et le troisième ordinateur 22 est configuré pour activer chaque élément vibrant 18 par palier de 20° d'angle de flexion du coude. Il est entendu que le palier peut avoir une valeur différente de 20°, par exemple 10°, 15°, 25°, 30°.

De préférence, le troisième ordinateur 22 est configuré pour déterminer la durée et la séquence des vibrations.

Par exemple, le troisième ordinateur 22 est configuré pour activer un élément vibrant 18 déterminé pendant une durée de 100 ms alternée avec un arrêt pendant une durée de 100 ms.

De préférence, l'intensité de vibration est adaptable pour améliorer le confort du sujet 10.

En variante, le dispositif de traitement 14 comprend un seul ordinateur, ou bien un smartphone, une télévision ou une tablette, ou toute combinaison envisageable de ces éléments. Par exemple, toutes les fonctions des premier, deuxième et troisième ordinateurs décrites ci-dessus sont aptes à être mises en œuvre par cet unique ordinateur.

Selon une autre variante, le dispositif de traitement est au moins partiellement embarqué dans le dispositif de mesure. Par exemple, la partie dispositif de traitement embarquée est configurée pour mettre en œuvre l'ensemble des fonctions des premier et deuxième ordinateurs décrites ci-dessus.

Une méthode de contrôle d'un membre d'un avatar virtuel par les activités myoélectriques d'un membre d'un sujet va maintenant être décrite. La méthode est mise en œuvre par le système 1.

Le dispositif de mesure 4 de signaux d'activité myoélectrique est placé sur un membre 8 du sujet 10 de sorte que les électrodes 6 soient en contact avec un premier et un deuxième muscles antagonistes de ce membre.

Par exemple, le sujet 10 est une personne amputée d'un membre. Le sujet 10 est par exemple amputé d'un bras et présente une amputation transhumérale, c'est-à-dire au-dessus du coude. Le dispositif de mesure 4 de signaux d'activité myoélectrique est placé autour du bras amputé du sujet 10 de sorte que les électrodes 6 soient en contact avec le biceps et le triceps, deux muscles antagonistes.

En variante, le sujet ne présente pas d'amputation et le dispositif de mesure de signaux d'activité myoélectrique est placé autour du bras du sujet de sorte que les électrodes soient en contact avec le biceps et le triceps. Le sujet non-amputé a le bras immobilisé dans un dispositif. Cela permet de reproduire la situation musculaire d'un sujet amputé, c'est-à-dire une contraction isométrique du biceps et du triceps caractérisée par une absence de déplacement.

La méthode comprend une première étape de calibration.

Les électrodes 6 acquièrent un premier et un deuxième signal d'activité myoélectrique brut de calibration, le premier et le deuxième signal d'activité myoélectrique brut de calibration résultant de contractions d'intensités variables du premier et du deuxième muscles antagonistes du membre 8 du sujet 10, respectivement, pendant une durée déterminée.

Le dispositif de mesure 4 de signaux d'activité myoélectrique communique à distance avec le dispositif de traitement 14 au moyen du dispositif de communication à distance 12. Dans l'exemple de la figure 1, le dispositif de mesure 4 communique à distance avec le premier ordinateur 20.

Avantageusement, la méthode comprend en outre l'identification de deux électrodes 6 du dispositif de mesure 4 permettant une acquisition maximale des signaux d'activité myoélectrique de chacun des deux muscles antagonistes. Pour ce faire, le sujet 10 effectue des contractions dissociées des deux muscles antagonistes portant le dispositif de mesure 4, par exemple le biceps et le triceps.

Chaque électrode 6 acquiert un signal d'activité myoélectrique brut de calibration qu'elle transmet au dispositif de traitement 14 au moyen du dispositif de communication à distance 12.

La fréquence d'acquisition des électrodes 6 est par exemple de 200 Hz.

Une fois les contractions réalisées, l'acquisition est arrêtée et le dispositif de traitement 14 sélectionne les deux électrodes 6 ayant acquis chacune une activité propre et importante lors de l'activation de l'un des muscles antagonistes et n'ayant pas ou peu acquis d'activités de co-contraction.

Les activités de co-contraction correspondent à une contraction simultanée des deux muscles antagonistes.

En particulier, le premier ordinateur 20 sélectionne les deux électrodes 6 permettant une acquisition maximale des signaux d'activité myoélectrique bruts de calibration de chacun des deux muscles antagonistes.

Après l'identification de deux électrodes 6 permettant une acquisition maximale, la méthode comprend la détermination, notamment par le dispositif de traitement 14, des activités myoélectriques minimales et maximales pour chacun des deux muscles antagonistes à partir du signal d'activité myoélectrique brut de calibration acquis.

En particulier, le premier ordinateur 20 détermine des activités myoélectriques minimales et maximales pour chacun des deux muscles antagonistes à partir du signal d'activité myoélectrique brut de calibration acquis.

Pour ce faire, le sujet 10 effectue par exemple la plus forte contraction musculaire possible du premier muscle, par exemple du biceps pendant une durée déterminée, puis relâche. La durée déterminée est comprise entre une seconde et trois secondes, de préférence égale à deux secondes. Puis le sujet 10 effectue la plus forte contraction musculaire possible du deuxième muscle, par exemple du triceps pendant une durée déterminée, puis relâche. La durée déterminée est par exemple deux secondes.

Un premier et un deuxième signal d'activité myoélectrique brut de calibration résultant de ces contractions est acquis par les électrodes 6.

Le dispositif de communication à distance 12 transmet le signal d'activité myoélectrique brut de calibration au dispositif de traitement 14.

En particulier, le dispositif de communication à distance 12 transmet le signal d'activité myoélectrique brut de calibration au premier ordinateur 20.

Le dispositif de traitement 14 met ensuite à valeur moyenne nulle le signal d'activité myoélectrique brut de calibration.

En particulier, le premier ordinateur 20 met à valeur moyenne nulle le signal d'activité myoélectrique brut de calibration.

Le dispositif de traitement 14 détermine la valeur absolue du signal d'activité myoélectrique brut de calibration mis à valeur moyenne nulle.

En particulier, le premier ordinateur 20 détermine la valeur absolue du signal d'activité myoélectrique brut de calibration mis à valeur moyenne nulle.

Le dispositif de traitement 14 filtre le signal en valeur absolue pour en obtenir l'enveloppe.

En particulier, le premier ordinateur 20 filtre le signal en valeur absolue pour en obtenir l'enveloppe.

Par exemple, le filtre utilisé est un filtre Butterworth de type passe-bas d'ordre 2 et de fréquence de coupure 1,5Hz.

A partir de l'enveloppe du signal filtré, le dispositif de traitement 14 détermine pour chacun des deux muscles antagonistes la valeur minimale et la valeur maximale de l'enveloppe du signal filtré.

En particulier, le premier ordinateur 20 détermine pour chacun des deux muscles antagonistes la valeur minimale et la valeur maximale de l'enveloppe du signal filtré.

La valeur minimale et la valeur maximale de l'enveloppe du signal filtré correspondent aux activités myoélectriques minimales et maximales.

Avantageusement, le dispositif de traitement 14 mémorise la valeur minimale et la valeur maximale de l'enveloppe du signal filtré.

Puis le dispositif de traitement 14 détermine un seuil d'activité myoélectrique, appelé offset $EMG_x$, en-dessous duquel la contraction du muscle n'est pas obligatoirement volontaire et ne doit pas être prise en considération afin d'éviter de perturber le mouvement produit. En effet, des valeurs situées en-deçà de ce seuil correspondent à une activité résiduelle des muscles suite à une fatigue musculaire, et pourraient perturber le mouvement.

En particulier, ce seuil est déterminé en tenant compte de la perception du sujet 10 et se calcule comme suit, pour chacun des deux muscles antagonistes :

$$offset\ EMG_x = perc_x * (EMG_{x\ max} - EMG_{x\ min}) + EMG_{x\ min}, \text{avec}$$

avec

offset $EMG_x$ le seuil d'activité myoélectrique ;
$perc_x$ le pourcentage sur l'étendue de l'activité musculaire ;
$EMG_{x\ max}$ la valeur maximale de l'enveloppe du signal filtré, et
$EMG_{x\ min}$ la valeur minimale de l'enveloppe du signal filtré.

Le pourcentage sur l'étendue de l'activité musculaire du sujet 10 est déterminé de manière à ce que la vitesse de déplacement du membre de l'avatar virtuel soit nulle lorsque le sujet se sent relâché.

Typiquement, ce pourcentage est compris entre 4% et 10%.

Ce pourcentage a un impact lorsque le sujet souhaite générer une vitesse de déplacement du membre de l'avatar virtuel mais aussi annuler cette vitesse.

Ce pourcentage est affiné pour chaque muscle.

La valeur de seuil d'activité myoélectrique est typiquement déterminée par défaut à 5% de l'étendue de l'activité musculaire.

Ensuite, ce seuil d'activité myoélectrique est typiquement testé et ajusté avec le sujet 10 afin d'évaluer s'il ne perturbe pas le contrôle de l'avatar virtuel 24.

De préférence, la valeur du seuil d'activité myoélectrique peut être ajustée à tout moment et notamment après plusieurs minutes de travail, où la fatigue musculaire peut se faire sentir et l'activité musculaire résiduelle devenir gênante.

En particulier, le premier ordinateur 20 détermine le seuil d'activité myoélectrique.

A partir de la détermination du seuil d'activité myoélectrique, le premier ordinateur 20 ne prendra en compte que les valeurs relevées entre le seuil déterminé et la valeur maximale de l'enveloppe du signal filtré.

Avantageusement, le dispositif de traitement 14 mémorise le seuil d'activité myoélectrique.

Puis le dispositif de traitement 14 normalise le seuil déterminé et la valeur maximale de l'enveloppe du signal filtré.

En particulier, le premier ordinateur 20 normalise le seuil déterminé et la valeur maximale de l'enveloppe du signal filtré.

Le premier ordinateur 20 attribue typiquement la valeur 0 au seuil d'activité myoélectrique précédemment calculé, et la valeur 1 à la valeur maximale de l'enveloppe du signal filtré.

Le dispositif de traitement 14 détermine, pour chaque muscle, un coefficient de conversion permettant de convertir toute valeur normalisée de l'enveloppe du signal filtré comprise entre le seuil déterminé et la valeur maximale de l'enveloppe du signal filtré en une composante de vitesse. Le coefficient de conversion est adapté au sujet pour chacun des muscles afin de rendre la perception de déplacement la plus fidèle possible. Le coefficient de conversion permet typiquement d'attribuer, pour chaque muscle, une composante de vitesse nulle lorsque la valeur normalisée de l'enveloppe du signal filtré est 0, et d'attribuer une composante de vitesse maximale lorsque la valeur normalisée de l'enveloppe du signal filtré est 1.

Avantageusement, le dispositif de traitement 14 mémorise le coefficient de conversion.

Une telle calibration des signaux d'activité myoélectrique relevés permet un réglage fin du contrôle de l'avatar virtuel 24 adapté au sujet 10 notamment lorsque les activités myoélectriques sont très inégales entre les deux muscles antagonistes.

Après cette phase de calibration, la méthode comprend une étape de mise en mouvement du membre de l'avatar virtuel 24.

Le système 1 est prêt pour acquérir de nouveaux signaux d'activité myoélectrique et les traiter.

Les deux électrodes 6 sélectionnées acquièrent un premier et un deuxième signal d'activité myoélectrique brut résultant de contractions des deux muscles antagonistes du membre 8 du sujet 10, pendant une durée déterminée.

Pour chacun des premier et deuxième signaux d'activité myoélectrique brut, le dispositif de communication à distance 12 transmet le signal d'activité myoélectrique brut au premier ordinateur 20.

Le premier ordinateur 20 met à valeur moyenne nulle le signal d'activité myoélectrique brut.

Le premier ordinateur 20 détermine la valeur absolue du signal d'activité myoélectrique brut mis à valeur moyenne nulle.

Le premier ordinateur 20 filtre le signal en valeur absolue pour en obtenir l'enveloppe.

Le premier ordinateur 20 normalise les valeurs de l'enveloppe du signal filtré, par exemple par une règle de trois à partir du seuil d'activité myoélectrique précédemment calculé et mémorisé, et de la valeur maximale de l'enveloppe du signal filtré précédemment déterminée et mémorisée pour obtenir une valeur comprise entre 0 et 1.

Le premier ordinateur 20 attribue typiquement, pour chaque muscle, à chaque valeur normalisée de l'enveloppe du signal filtré, une composante de vitesse comprise entre la composante de vitesse nulle et la composante de vitesse maximale, par application du coefficient précédemment déterminé et mémorisé.

Le premier ordinateur 20 détermine une vitesse et une direction de déplacement d'un membre de l'avatar virtuel 24 affiché par le deuxième ordinateur 21 par soustraction des composantes de vitesse obtenues pour chacun des muscles.

Par exemple, dans le cas de la rotation d'un coude, la soustraction des valeurs de vitesse permet de déterminer une vitesse de flexion ou d'extension d'un coude d'un avatar virtuel 24 affiché par le deuxième ordinateur 21.

Le premier ordinateur 20 transmet une commande de mouvement au deuxième ordinateur 21.

La commande de mouvement comprend typiquement la vitesse et la direction de déplacement du membre de l'avatar virtuel 24 déterminées.

Le deuxième ordinateur 21 reçoit la commande de mouvement transmise par le premier ordinateur 20.

Le dispositif d'affichage 23 du deuxième ordinateur 21 affiche l'avatar virtuel 24 représentant au moins un membre correspondant au mouvement qui doit être commandé par les activités myoélectriques. Il peut s'agir du bras ou d'une jambe, d'un corps entier ou un d'hémicorps en fonction de l'application choisie.

Le membre de l'avatar virtuel 24 se déplace en fonction de la commande de mouvement transmise par le premier ordinateur 20.

Par exemple, l'arrêt des contractions musculaires entraîne l'arrêt du mouvement, le positionnement du coude de l'avatar virtuel 24 est alors en position fixe qui correspond à la dernière position atteinte.

Ainsi, lorsque le sujet 10 contracte au moins l'un des muscles sur lesquels les électrodes 6 sont placées, l'avatar virtuel 24 fléchit ou étend son coude en fonction des réglages effectués adaptés au sujet.

Selon des variantes, la vitesse calculée est une vitesse d'ouverture ou de fermeture de main d'un avatar virtuel, ou une vitesse de rotation de poignet d'un avatar virtuel, ou une vitesse de flexion/extension de genou ou de la cheville d'un avatar virtuel, ou toute autre option envisageable.

Le sujet 10 a ainsi un retour visuel des contractions qu'il effectue avec les deux muscles antagonistes, par l'avatar virtuel 24 dont les mouvements sont adaptés aux capacités de contraction musculaire du sujet. L'apprentissage de la dissociation musculaire et le travail fin des contractions musculaires sont ainsi améliorés.

Dans un mode de réalisation avantageux de la méthode, après avoir déterminé une vitesse de déplacement et une direction de déplacement du membre de l'avatar virtuel 24 par l'acquisition des premier et deuxième signaux d'activité myoélectrique, la méthode comprend l'activation du dispositif de retour sensoriel 16, notamment par le dispositif de traitement 14.

Les éléments vibrants 18 du dispositif de retour sensoriel 16 sont disposés sur le membre 8 du sujet 10 comprenant les deux muscles antagonistes dont l'activité myoélectrique est mesurée.

Les éléments vibrants 18 sont par exemple au nombre de seize et disposés en deux bandeaux de huit de part et d'autre des électrodes 6.

L'activation des éléments vibrants 18 est par exemple réalisée de la façon suivante.

Pour chaque signal d'activité myoélectrique acquis par les électrodes 6, le premier ordinateur 20 détermine une valeur instantanée d'un paramètre sensoriel, par exemple une position instantanée du membre de l'avatar virtuel 24 à partir de la vitesse et de la direction de déplacement déterminées précédemment.

Le premier ordinateur 20 envoie au troisième ordinateur 22 la valeur instantanée du paramètre sensoriel du membre de l'avatar virtuel 24.

Le troisième ordinateur 22 détermine des fourchettes de valeurs du paramètre sensoriel du membre de l'avatar virtuel 24 à associer à au moins un élément vibrant 18.

Le troisième ordinateur 22 active typiquement au moins un élément 18 correspondant à une fourchette de valeurs du paramètre sensoriel préalablement déterminée.

En particulier, le programme de lecture du troisième ordinateur 22 fait la correspondance entre la valeur instantanée du paramètre sensoriel et l'activation de l'élément vibrant 18 correspondant.

De préférence, le troisième ordinateur 22 active les éléments vibrants 18 de manière sélective et discrète.

Par exemple, le troisième ordinateur 22 détermine le nombre et la position des éléments vibrants 18 à activer en fonction de fourchettes de valeurs du paramètre sensoriel.

De préférence, dans chaque fourchette de valeurs du paramètre sensoriel.

De préférence encore, à une fourchette de valeurs du paramètre sensoriel est attribué un unique élément vibrant 18 distinct pour chaque fourchette de valeurs du paramètre sensoriel.

Selon l'exemple où le paramètre sensoriel est l'angle de flexion du coude, lorsque le sujet 10 contracte le biceps et/ou le triceps, les électrodes 6 acquièrent un premier et un deuxième signal d'activité myoélectrique brut résultant de contractions du biceps et du triceps, respectivement, le dispositif de communication à distance 12 transmet le premier et le deuxième signal d'activité myoélectrique au premier ordinateur 20, le premier ordinateur 20 traite le premier et le deuxième signal d'activité myoélectrique et transmet au deuxième ordinateur 21 une commande de déplacement du bras de l'avatar virtuel 24 et au troisième ordinateur 22 une position instantanée du bras de l'avatar virtuel 24, le deuxième ordinateur 21 affiche le déplacement du bras de l'avatar virtuel 24 et le troisième ordinateur 22 active chaque élément vibrant 18 par palier de 20° d'angle de flexion du coude. Il est entendu que le palier peut avoir une valeur différente de 20°, par exemple 10°, 15°, 25°, 30°.

Cela permet un contrôle le plus naturel possible c'est-à-dire à la fois rapide dans l'exécution du mouvement mais aussi précis pour permettre un arrêt sur une cible sans oscillation.

De préférence, le troisième ordinateur 22 détermine la durée et la séquence des vibrations pour chaque position instantanée.

Par exemple, pour une position instantanée donnée, le troisième ordinateur 22 active un élément vibrant déterminé 18 pendant une durée de 100 ms alternée avec un arrêt pendant une durée de 100 ms.

De préférence, l'intensité de vibration est adaptable pour améliorer le confort du sujet 10.

Par exemple, un premier niveau d'intensité est défini par le ressenti du sujet d'une première vibration lorsque l'intensité de vibration est augmentée depuis une absence de vibration. Un second niveau d'intensité est défini par un passage depuis le ressenti du sujet d'une vibration à l'absence de ressenti de vibration lorsque l'intensité de vibration est diminuée depuis une forte vibration.

Un niveau d'intensité de vibration suffisant pour stimuler le sujet est calculé à l'aide de la moyenne entre le premier et le second niveau d'intensité de vibration.

Selon un autre exemple, l'intensité de vibration varie suivant les activités myoélectriques relevées. Dans le cas d'une force de serrage d'une main de l'avatar virtuel 24, plus la force est élevée et plus l'intensité de vibration appliquée est élevée.

Le retour sensoriel par vibration permet d'ajouter une information sensorielle congruente à la vision en réduisant la charge attentionnelle du sujet, comme cela est illustré dans l'exemple ci-dessous.

Un exemple de mise en oeuvre de la méthode selon l'invention va maintenant être décrit.

## EXEMPLE

*Matériel et participants*

Les participants comprennent des sujets amputés d'un bras en transhuméral, c'est-à-dire au-dessus du coude, et des sujets non-amputés, dits sains.

L'outil utilisé pour récupérer les activités électriques du biceps et du triceps est le MyoArm Band ® de la société Thalmic. C'est un bracelet comprenant 8 électrodes de surface, initialement conçues pour fonctionner à l'aide d'un algorithme de patron d'activités musculaires au niveau de l'avant-bras. Il est utilisé ici pour récupérer les activités musculaires électriques du biceps et du triceps au niveau du bras des participants.

Un bracelet de 6 vibreurs de 7 mm de diamètre et de 2 mm d'épaisseur est utilisé pour envoyer un retour sensoriel vibrotactile au participant.

Trois ordinateurs sont connectés entre eux avec, pour chacun, un rôle bien précis. L'ordinateur principal, l'ordinateur maître, est le support des programmes de l'ensemble du protocole pour le déroulement des différents exercices, et a également pour rôle de (1) réceptionner les activités myoélectriques envoyées depuis le MyoArm Band par Bluetooth, (2) les traiter, (3) envoyer au deuxième ordinateur la commande de mouvement traduite en une vitesse (issue de ce traitement) pour que le coude d'un avatar virtuel affiché à l'écran du deuxième ordinateur bouge et (4) envoyer la position instantanée calculée depuis les activités myoélectriques au troisième ordinateur qui se charge de l'activation des vibreurs. Les échanges entre l'ordinateur maître et les deux autres ordinateurs se font à l'aide d'un switch paramétré pour interagir en réseau local.

Le deuxième ordinateur est dédié à l'interface visuelle et placé en face du participant. Le logiciel Animatlab est utilisé sur cet ordinateur pour visualiser en temps réel le mouvement de l'avatar. De plus, pendant les exercices, toutes les consignes sont affichées sur cet ordinateur. Les participants n'ont qu'à se focaliser sur l'écran et suivre les différentes instructions.

Enfin, le troisième ordinateur (pi-top) a pour rôle de gérer l'activation des vibreurs. Il reçoit les instructions de l'ordinateur maître lui indiquant la position calculée depuis les activités myoélectriques. Un programme de lecture sur le pi-top fait la correspondance entre la position et l'activation du vibreur correspondant. Cet ordinateur est constitué d'un Raspberry Pi3 auquel est branché le bracelet de vibreurs.

*Calibration*

Avant tout début de test, chaque participant passe par une phase nécessaire de calibration. Lors de cette phase, les activités musculaires provenant des 8 électrodes du bracelet sont affichées sur une interface graphique. Il suffit de mettre le bracelet et de regarder les activités musculaires enregistrées par les électrodes directement. La fréquence d'acquisition du MyoArm Band est de 200Hz. L'activité brute ainsi que le tracé du signal filtré sont visibles. Durant cette phase il est demandé aux participants d'effectuer des contractions dissociées du biceps et du triceps.

Cela permet d'identifier les deux électrodes avec lesquelles le signal est le plus clair pour la suite de l'expérimentation. Une fois les contractions réalisées, l'acquisition est arrêtée et les deux électrodes qui avaient (i) une activité propre et importante lors de l'activation du biceps ou du triceps et (ii) qui n'avaient pas ou peu d'activités de co-contraction sont sélectionnées.

Une fois les électrodes sélectionnées, l'activité myoélectrique minimale et maximale est déterminée sur chacune des voies des électrodes. Pour cela, il est demandé aux participants d'effectuer une contraction quasi-maximale pendant 2 sec du biceps, puis un relâchement et une contraction de 2 sec du triceps.

Un avatar avec le bras droit ou le bras gauche est disponible en fonction de la latéralité de l'amputation des participants. Ensuite, le signal est normalisé. Un seuil d'activité musculaire est défini (souvent entre 5% et 7,5 % de l'étendue du signal normalisé) en deçà duquel aucun mouvement ne serait engendré. Cela permet de prévenir l'apparition de l'activité résiduelle des muscles suite à une fatigue musculaire qui pourrait mettre en mouvement l'avatar sans réelle volonté du sujet. Ce seuil est réglable en cours d'expérience donnant une certaine marge de manoeuvre pour ajuster le seuil en fonction de la condition des participants. Enfin, un gain est mis en place entre l'activité musculaire et la vitesse de rotation du coude. Cela permet un réglage fin du contrôle de l'avatar notamment lorsque les activités musculaires sont très inégales.

Des repères spatiaux sont définis afin d'activer les vibrations à raison d'une stimulation rapide faite d'alternance (activée pendant 100ms puis arrêtée pendant 100ms et ainsi de suite). Après une présence (dans le cas précis de plus de 2 secondes) à l'un de ces repères, le temps d'arrêt de stimulation est augmenté afin de moins solliciter le participant mais tout en continuant de l'informer toutes les 500ms par une activation de 100ms.

Lorsque les participants contractent leurs muscles, le bras de l'avatar virtuel bouge sur la base des données issues de la calibration et les vibreurs s'activent par palier de 20° d'angle.

*Expérimentation*

Des mouvements d'allers-retours entre deux cibles étaient demandés aux participants afin d'évaluer la stabilité du contrôle dans le temps. L'écran du deuxième ordinateur affichait les 2 cibles représentant l'amplitude du mouvement dans lequel se déplacer avec le bras de l'avatar en regard de la cible de départ. Les amplitudes de mouvement allaient de 20° à 100°. Cet exercice d'entraînement comprenait une série de 10 mouvements à réaliser en présence du retour sensoriel visuel, puis 10 mouvements en présence des retours sensoriels visuel et vibratoire et 10 mouvements avec la vibration seule. Lors de la réalisation du mouvement, il était demandé aux sujets de marquer un temps de pause sur la cible à atteindre avant de repartir vers l'autre cible. Un son notifiait le caractère stable de la position de l'avatar lorsqu'il n'avait pas bougé pendant 500 ms. Cependant, il ne notifiait pas le fait que les sujets étaient sur la bonne cible. La validation de la cible ne pouvait donc se faire qu'avec la vision et/ou la vibration. A la fin de chaque essai le tracé du mouvement était montré aux participants. Cela leur permettait de juger de la vitesse de réalisation de mouvement et de la précision sur l'ensemble de l'essai. La durée pour la réalisation de l'essai était proportionnelle à l'amplitude des allers-retours à réaliser et s'étendait d'environ 20 secondes à 45 secondes pour l'essai avec 100° d'amplitude. Une fois les 3 séries de 10 mouvements terminées, les participants enchainaient sur la phase de test. Cet exercice avait une durée approximative de 45 minutes.

Phase de test : La phase de test comprenait un total de 8 blocs de 3 essais chacun, avec un 1 essai par type de retour sensoriel. Tous les deux blocs (soit tous les 6 mouvements) un essai sans aucun retour sensoriel était réalisé. Cet essai servait de ligne de base pour évaluer les performances avec les autres types de retour sensoriel. Un total de 28 mouvements était donc réalisé dans cette phase de test. De manière similaire à la phase d'entrainement, un temps de pause sur les cibles à atteindre était demandé. L'objectif n'était pas de faire le plus d'allers-retours possible mais d'être précis et sur les bonnes cibles. En fin d'essai, le tracé du mouvement réalisé était affiché à l'écran pour montrer aux participants la qualité de leur prestation. Cette phase de test avait une durée approximative de 40 minutes.

*Résultats*

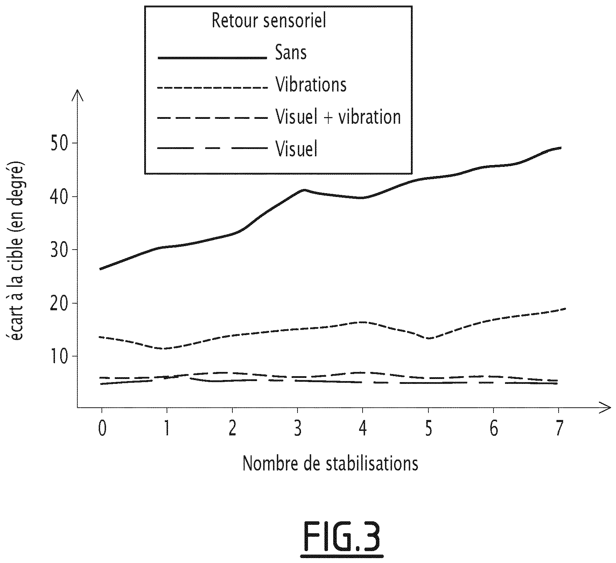

La figure 3 illustre le comportement des sujets non-amputés et représente la distribution des écarts à la cible pour chaque mouvement au cours des essais d'allers-retours représenté pour les quatre types de retour sensoriel. L'axe des abscisses représente le nombre de stabilisations réalisées par les sujets lors des mouvements d'aller-retour. L'axe des ordonnées représente l'écart à cette cible. Un aller-retour équivaut à deux itérations. Les traits pleins ou pointillés représentent la moyenne des écarts à la cible pour l'ensemble des essais utilisant le même type de retour sensoriel.

Les tracés montrent que pour les retours sensoriels incluant la vision, les sujets restent en moyenne très précis avec un écart inférieur à 10° pour plus de 3 allers-retours. Lorsque l'on regarde le tracé de la condition avec le retour sensoriel vibrotactile seul, celui-ci est stable au cours du temps avec des valeurs supérieures aux tracés incluant la vision mais en deçà de la limite de 20° qui correspond à la discrétisation de 2 vibreurs. On observe clairement que la dernière condition sans aucun retour sensoriel est différente des 3 autres conditions avec une erreur qui augmente au cours du temps. Cela atteste que les sujets n'ayant aucun retour sensoriel sur la réalisation de leur action perdaient leurs repères et s'écartaient des cibles au cours du temps. Ce tracé illustre donc l'effet positif du retour sensoriel vibrotactile dans le sens où il permet aux sujets de se situer dans l'espace. L'information transmise par les vibrations est donc correctement utilisée et efficiente.

## Revendications

1.   Méthode de contrôle d'un membre d'un avatar virtuel (24) par les activités myoélectriques d'un membre (8) d'un sujet (10), la méthode de contrôle étant mise en oeuvre par un système de contrôle d'un membre d'un avatar virtuel (24) comprenant un dispositif de mesure (4) de signaux d'activité myoélectrique et un dispositif de traitement (14) des activités myoélectriques comprenant au moins un dispositif d'affichage (23) configuré pour afficher l'avatar virtuel (24), la méthode de contrôle comprenant une première étape de calibration comprenant:

- l'acquisition d'un premier et d'un deuxième signal d'activité myoélectrique brut de calibration par le dispositif de mesure (4) de signaux d'activité myoélectrique,
le premier et le deuxième signal d'activité myoélectrique brut de calibration résultant de contractions d'intensités variables d'un premier et d'un deuxième muscles antagonistes du membre (8) du sujet (10) respectivement, pendant une durée déterminée, puis :

- la détermination de l'enveloppe de chacun du premier et du deuxième signal par le dispositif de traitement (14),
- la détermination des activités myoélectriques minimales et maximales à partir de l'enveloppe de chacun du premier et du deuxième signal par le dispositif de traitement (14), puis pour chacun des deux muscles antagonistes:
- la détermination d'un seuil d'activité myoélectrique par le dispositif de traitement (14),
- la normalisation de l'activité myoélectrique maximale et du seuil d'activité myoélectrique par le dispositif de traitement (14),
- la détermination d'un coefficient de conversion de l'activité myoélectrique normalisée en une composante de vitesse de déplacement d'un membre de l'avatar virtuel (24) par le dispositif de traitement (14), et

la méthode de contrôle comprenant une deuxième étape de mise en mouvement du membre de l'avatar virtuel (24) comprenant:

- l'acquisition d'un premier et d'un deuxième signal d'activité myoélectrique brut par le dispositif de mesure (4) de signaux d'activité myoélectrique, le premier et le deuxième signal d'activité myoélectrique brut résultant de contractions des deux muscles antagonistes du membre (8) du sujet (10), pendant une durée déterminée, puis pour chacun du premier et du deuxième signal d'activité myoélectrique brut :

- la normalisation de l'activité myoélectrique issue du signal par le dispositif de traitement (14),
- la conversion de l'activité myoélectrique normalisée en une composante de vitesse de déplacement d'un membre d'un avatar virtuel (24) par application du coefficient de conversion par le dispositif de traitement (14), - la détermination d'une vitesse et d'une direction de déplacement du membre de l'avatar virtuel (24) par soustraction des composantes de vitesse de déplacement obtenues pour chacun des muscles par le dispositif de traitement (14), et

- le déplacement du membre de l'avatar virtuel (24) selon la vitesse et la direction de déplacement déterminées par le dispositif de traitement (14).

2. Méthode de contrôle selon la revendication 1, dans laquelle, simultanément à l'étape de déplacement du membre de l'avatar virtuel (24) selon la vitesse et la direction de déplacement déterminées, la méthode comprend l'activation d'un dispositif de retour sensoriel (16) disposé sur le membre (8) du sujet (10), l'activation du dispositif de retour sensoriel (16) étant effectuée de manière sélective et discrète en fonction de fourchettes de valeurs prédéterminées d'un paramètre sensoriel.

3. Méthode de contrôle selon la revendication 2, dans laquelle le paramètre sensoriel est choisi parmi un angle de flexion d'un coude de l'avatar virtuel (24), un angle d'élévation antérieure d'une épaule de l'avatar virtuel (24), un angle d'abduction/adduction de l'épaule de l'avatar virtuel (24), un angle de rotation médiale ou latérale de l'épaule de l'avatar virtuel (24), un angle de flexion d'un poignet de l'avatar virtuel (24), un angle de rotation radial ou ulnaire de l'avatar virtuel (24), un degré d'ouverture d'une main de l'avatar virtuel (24), un degré d'ouverture d'une prise en pince digitale impliquant le pouce et un ou plusieurs autres doigts de la main de l'avatar virtuel (24), une force de serrage d'une main de l'avatar virtuel (24), un angle de flexion d'un genou de l'avatar virtuel (24), un angle de flexion de la cheville de l'avatar virtuel (24), une vitesse de rotation de l'un des mouvements mentionnés dans la présente liste, ou toute autre modalité sensorielle susceptible d'être substituée par des vibrations, telle que la chaleur, ou la configuration d'une prothèse myoélectrique (11) du sujet (10).

4. Méthode de contrôle selon l'une quelconque des revendications 1 à 3, dans laquelle le dispositif de mesure (4) de signaux d'activité myoélectrique comprend une pluralité d'électrodes (6), la méthode comprenant la sélection de deux électrodes (6) parmi la pluralité d'électrodes (6) permettant une acquisition maximale des signaux d'activité myoélectrique de chacun des deux muscles antagonistes.

5. Méthode de contrôle selon l'une des revendications 1 à 4, dans laquelle la détermination des activités myoélectriques minimales et maximales à partir de l'enveloppe du signal comprend : la mise à valeur moyenne nulle du signal d'activité myoélectrique brut de calibration, la détermination de la valeur absolue du signal d'activité myoélectrique brut de calibration mis à valeur moyenne nulle, le filtrage du signal en valeur absolue pour en obtenir l'enveloppe, et à partir de l'enveloppe du signal filtré, la détermination de la valeur minimale et la valeur maximale de l'enveloppe du signal filtré.

6. Système de contrôle d'un membre d'un avatar virtuel (24) par les activités myoélectriques d'un membre (8) d'un sujet (10), le système comprenant :

- un dispositif de mesure (4) de signaux d'activité myoélectrique résultant de contractions d'un premier et d'un deuxième muscles antagonistes du membre (8) du sujet (10), propre à acquérir un premier et un deuxième signal d'activité myoélectrique brut de calibration résultant de contractions d'intensités variables d'un premier et d'un deuxième muscles antagonistes du membre (8) du sujet (10) respectivement, pendant une durée déterminée, et un premier et un deuxième signal d'activité myoélectrique brut résultant de contractions des deux muscles antagonistes du membre (8) du sujet (10), pendant une durée déterminée,
- un dispositif de traitement (14) des activités myoélectriques comprenant au moins un dispositif d'affichage (23) configuré pour afficher l'avatar virtuel (24), le dispositif de traitement (14) étant configuré pour mettre en œuvre la méthode de contrôle d'un membre d'un avatar virtuel (24) par les activités myoélectriques d'un membre (8) d'un sujet (10) selon l'une quelconque des revendications précédentes.

7. Système selon la revendication 6, dans lequel le dispositif de mesure (4) comprend une pluralité d'électrodes (6) et se présente sous forme d'un bracelet.

8. Système selon la revendication 6 ou 7, comprenant en outre un dispositif de retour sensoriel (16).

9. Système selon la revendication 8, dans lequel le dispositif de retour sensoriel (16) comprend une pluralité d'éléments vibrants (18).

**10.** Système selon la revendication 8 ou 9, dans lequel le dispositif de traitement (14) est configuré pour activer le dispositif de retour sensoriel (16) de manière sélective et discrète en fonction de fourchettes de valeurs prédéterminées d'un paramètre sensoriel.

**11.** Système selon l'une quelconque des revendications 6 à 10, dans lequel le dispositif de mesure (4) de signaux d'activité myoélectrique comprend un dispositif de communication à distance (12) configuré pour communiquer à distance avec le dispositif de traitement (14).

**Patentansprüche**

**1.** Verfahren zur Steuerung einer Gliedmaße eines virtuellen Avatars (24) durch die myoelektrischen Aktivitäten einer Gliedmaße (8) einer Person (10), wobei das Steuerungsverfahren durch ein Steuerungssystem für eine Gliedmaße eines virtuellen Avatars (24) implementiert wird, das eine Messvorrichtung (4) für myoelektrische Aktivitätssignale und eine Verarbeitungsvorrichtung (14) für die myoelektrischen Aktivitäten umfasst, die mindestens eine Anzeigevorrichtung (23) umfasst, die dazu eingerichtet ist, den virtuellen Avatar (24) anzuzeigen, wobei das Steuerungsverfahren einen ersten Kalibrierschritt umfasst, der Folgendes umfasst:

- Erfassen eines ersten und eines zweiten myoelektrischen Rohkalibrierungsaktivitätssignals durch die Messvorrichtung (4) für myoelektrische Aktivitätssignale, wobei sich das erste und das zweite myoelektrische Rohkalibrierungsaktivitätssignal aus Kontraktionen unterschiedlicher Intensität eines ersten bzw. eines zweiten antagonistischen Muskels der Gliedmaße (8) der Person (10) für eine bestimmte Dauer ergeben, dann:

- Bestimmen der Hüllkurve jeweils des ersten und des zweiten Signals durch die Verarbeitungsvorrichtung (14),
- Bestimmen der minimalen und der maximalen myoelektrischen Aktivität aus der Hüllkurve jeweils des ersten und des zweiten Signals durch die Verarbeitungsvorrichtung (14) und dann für jeden der beiden antagonistischen Muskeln:

- Bestimmen eines Schwellenwerts der myoelektrischen Aktivität durch die Verarbeitungsvorrichtung (14),
- Normalisieren der maximalen myoelektrischen Aktivität und des Schwellenwerts der myoelektrischen Aktivität durch die Verarbeitungsvorrichtung (14),
- Bestimmen eines Umwandlungskoeffizienten der normalisierten myoelektrischen Aktivität in eine Verschiebungsgeschwindigkeitskomponente einer Gliedmaße des virtuellen Avatars (24) durch die Verarbeitungsvorrichtung (14), und

wobei das Steuerungsverfahren einen zweiten Schritt zum Bewegen der Gliedmaße des virtuellen Avatars (24) umfasst, der Folgendes umfasst:

- Erfassen eines ersten und eines zweiten myoelektrischen Rohkalibrierungsaktivitätssignals durch die Messvorrichtung (4) für myoelektrische Aktivitätssignale, wobei sich das erste und das zweite myoelektrische Rohkalibrierungsaktivitätssignal aus Kontraktionen der beiden antagonistischen Muskeln der Gliedmaße (8) der Person (10) für eine bestimmte Dauer ergeben, und dann für jedes des ersten und des zweiten myoelektrischen Rohaktivitätssignals:

- Normalisieren der myoelektrischen Aktivität aus dem Signal durch die Verarbeitungsvorrichtung (14),
- Umwandeln der normalisierten myoelektrischen Aktivität in eine Bewegungsgeschwindigkeitskomponente einer Gliedmaße eines virtuellen Avatars (24) durch Anwendung des Umwandlungskoeffizienten durch die Verarbeitungsvorrichtung (14), - Bestimmen einer Geschwindigkeit und einer Bewegungsrichtung der Gliedmaße des virtuellen Avatars (24) durch Subtraktion der Bewegungsgeschwindigkeitskomponenten, die für jeden der Muskeln durch die Verarbeitungsvorrichtung (14) erhalten werden, und
- Bewegen der Gliedmaße des virtuellen Avatars (24) entsprechend der von der Verarbeitungsvorrichtung (14) bestimmten Geschwindigkeit und Bewegungsrichtung.

**2.** Steuerungsverfahren nach Anspruch 1, wobei das Verfahren gleichzeitig mit dem Schritt des Bewegens der Gliedmaße des virtuellen Avatars (24) entsprechend der bestimmten Geschwindigkeit und der bestimmten Bewegungsrichtung das Aktivieren einer an der Gliedmaße (8) der Person (10) angeordneten sensorischen Rückmeldungsvorrichtung (16) umfasst, wobei das Aktivieren der sensorischen Rückmeldungsvorrichtung (16) selektiv und diskret in Abhängigkeit von vorbestimmten Wertebereichen eines sensorischen Parameters erfolgt.

3. Steuerungsverfahren nach Anspruch 2, wobei der sensorische Parameter aus einem Beugewinkel eines Ellenbogens des virtuellen Avatars (24), einem vorderen Höhenwinkel einer Schulter des virtuellen Avatars (24), einem Abduktions-/Adduktionswinkel der Schulter des virtuellen Avatars (24), einem medialen oder lateralen Rotationswinkel der Schulter des virtuellen Avatars (24), einem Beugewinkel eines Handgelenks des virtuellen Avatars (24), einem radialen oder ulnaren Rotationswinkel des virtuellen Avatars (24), einem Öffnungsgrad einer Hand des virtuellen Avatars (24), einem Öffnungsgrad eines Fingergreifers, der den Daumen und einen oder mehrere andere Finger der Hand des virtuellen Avatars (24) einbezieht, einer Klemmkraft einer Hand des virtuellen Avatars (24), einem Beugewinkel eines Knies des virtuellen Avatars (24), einem Beugewinkel des Sprunggelenks des virtuellen Avatars (24), einer Rotationsgeschwindigkeit einer der in der vorliegenden Liste genannten Bewegungen oder einer anderen sensorischen Modalität, die durch Vibrationen ersetzt werden kann, wie z. B. Wärme oder die Konfiguration einer myoelektrischen Prothese (11) der Person (10), ausgewählt ist.

4. Steuerungsverfahren nach einem der Ansprüche 1 bis 3, wobei die Messvorrichtung (4) für myoelektrische Aktivitätssignale eine Vielzahl von Elektroden (6) umfasst, wobei das Verfahren die Auswahl von zwei Elektroden (6) aus der Vielzahl von Elektroden (6) umfasst, die eine maximale Erfassung der myoelektrischen Aktivitätssignale von jedem der beiden antagonistischen Muskeln ermöglichen.

5. Steuerungsverfahren nach einem der Ansprüche 1 bis 4, wobei das Bestimmen der minimalen und der maximalen myoelektrischen Aktivität aus der Hüllkurve des Signals Folgendes umfasst: Einstellen des myoelektrischen Rohkalibrierungsaktivitätssignals auf einen Mittelwert von Null, Bestimmen des Absolutwerts des auf einen Mittelwert von Null eingestellten myoelektrischen Rohkalibrierungsaktivitätssignals, Filtern des Signals nach dem Absolutwert, um die Hüllkurve zu erhalten, und ausgehend von der Hüllkurve des gefilterten Signals das Bestimmen des minimalen Werts und des maximalen Werts der Hüllkurve des gefilterten Signals.

6. System zur Steuerung einer Gliedmaße eines virtuellen Avatars (24) durch die myoelektrischen Aktivitäten einer Gliedmaße (8) einer Person (10), wobei das System Folgendes umfasst:

   - eine Messvorrichtung (4) für myoelektrische Aktivitätssignale, die sich aus Kontraktionen eines ersten und eines zweiten antagonistischen Muskels der Gliedmaße (8) der Person (10) ergeben, die geeignet ist, einen ersten und einen zweiten myoelektrischen Rohkalibrierungsaktivitätssignals, die sich aus Kontraktionen unterschiedlicher Intensität eines ersten und eines zweiten antagonistischen Muskels der Gliedmaße (8) der Person (10) jeweils für eine bestimmte Dauer ergeben, und einen ersten und einen zweiten myoelektrischen Rohaktivitätssignals zu erfassen, die sich aus Kontraktionen der beiden antagonistischen Muskeln der Gliedmaße (8) der Person (10) für eine bestimmte Dauer ergeben,
   - eine Verarbeitungsvorrichtung (14) für die myoelektrischen Aktivitäten, die mindestens eine Anzeigevorrichtung (23) umfasst, die dazu eingerichtet ist, den virtuellen Avatar (24) anzuzeigen, wobei die Verarbeitungsvorrichtung (14) dazu eingerichtet ist, das Verfahren zur Steuerung einer Gliedmaße eines virtuellen Avatars (24) durch die myoelektrischen Aktivitäten einer Gliedmaße (8) einer Person (10) nach einem der vorhergehenden Ansprüche zu implementieren.

7. System nach Anspruch 6, wobei die Messvorrichtung (4) eine Vielzahl von Elektroden (6) umfasst und in Form eines Armbands vorliegt.

8. System nach Anspruch 6 oder 7, das ferner eine sensorische Rückmeldungsvorrichtung (16) umfasst.

9. System nach Anspruch 8, wobei die sensorische Rückmeldungsvorrichtung (16) eine Vielzah von Schwingungselementen (18) umfasst.

10. System nach Anspruch 8 oder 9, wobei die Verarbeitungsvorrichtung (14) dazu eingerichtet ist, die sensorische Rückmeldungsvorrichtung (16) selektiv und diskret in Abhängigkeit von vorbestimmten Wertebereichen eines sensorischen Parameters zu aktivieren.

11. System nach einem der Ansprüche 6 bis 10, wobei die Messvorrichtung (4) für myoelektrische Aktivitätssignale eine Fernkommunikationsvorrichtung (12) umfasst, die dazu eingerichtet ist, mit der Verarbeitungsvorrichtung (14) über die Ferne zu kommunizieren.

## Claims

1. Method for controlling a limb of a virtual avatar (24) by the myoelectric activities of a limb (8) of a subject (10), the method for controlling being implemented by a system for controlling a limb of a virtual avatar (24) comprising a device (4) for measuring myoelectric activity signals and a processing device (14) for processing the myoelectric activities comprising at least one display device (23) configured for displaying the virtual avatar (24), the method for controlling comprising a first calibration step comprising:

   - acquiring first and second raw calibration myoelectric activity signals by the device (4) for measuring myoelectric activity signals, the first and second raw calibration myoelectric activity signals resulting from contractions of variable intensities of first and second antagonistic muscles of the limb (8) of the subject (10) respectively, during a given period, and then:

      - determining the envelope of each of the first and second signals by the processing device (14),
      - determining the minimum and maximum myoelectric activities from the envelope of each of the first and second signals by the processing device (14), then, for each of the two antagonistic muscles:

         - determining a myoelectric activity threshold by the processing device (14),
         - normalizing the maximum myoelectric activity and the myoelectric activity threshold by the processing device (14),
         - determining a coefficient of conversion of the normalized myoelectric activity into a component of speed of movement of a limb of the virtual avatar (24) by the processing device (14), and

   the method for controlling comprising a second step of moving the limb of the virtual avatar (24) comprising:

      - acquiring first and second raw myoelectric activity signal by the device (4) for measuring myoelectric activity signals, the first and second raw myoelectric activity signals resulting from contractions of the two antagonistic muscles of the limb (8) of the subject (10), during a given period, and then, for each of the first and second raw myoelectric activity signals:

         - normalizing the myoelectric activity resulting from the signal by the processing device (14),
         - converting the normalized myoelectric activity into a component of speed of movement of a limb of a virtual avatar (24) by applying the coefficient of conversion by the processing device (14),
         - determining a speed and a direction of movement of the limb of the virtual avatar (24) by subtracting the movement speed components obtained for each of the muscles by the processing device (14), and
         - moving the limb of the virtual avatar (24) with the speed and direction of movement determined by the processing device (14).

2. Method for controlling according to claim 1, wherein, simultaneously with the step of moving the limb of the virtual avatar (24) with the determined speed and direction of movement, the method comprises the activation of a sensory feedback device (16) disposed on the limb (8) of the subject (10), the sensory feedback device (16) being activated selectively and discretely according to predetermined ranges of values of a sensory parameter.

3. Method for controlling according to claim 2, wherein the sensory parameter is selected from a bending angle of an elbow of the virtual avatar (24), an anterior elevation angle of a shoulder of the virtual avatar (24) an abduction/adduction angle of the shoulder of the virtual avatar (24), a medial or lateral rotation angle of the shoulder of the virtual avatar (24), a bending angle of a wrist of the virtual avatar (24), a radial or ulnar rotation angle of the virtual avatar (24), a degree of opening of a hand of the virtual avatar (24), a degree of opening of a finger grip involving the thumb and one or more other fingers of the hand of the virtual avatar (24), a gripping force of a hand of the virtual avatar (24), a bending angle of a knee of the virtual avatar (24), a bending angle of the ankle of the virtual avatar (24), a speed of rotation of one of the movements mentioned in the present list, or any other sensory modality able to be replaced by vibrations, such as the warmth or configuration of a myoelectric prosthesis (11) of the subject (10).

4. Method for controlling according to any one of claims 1 to 3, wherein the device (4) for measuring myoelectric activity signals comprises a plurality of electrodes (6), the method comprising selecting two electrodes (6) from the plurality of electrodes (6) enabling a maximum acquisition of the myoelectric activity signals of each of the two antagonistic muscles.

5. Method for controlling according to one of claims 1 to 4, wherein determining the minimum and maximum myoelectric activities from the envelope of the signal comprises: setting the raw calibration myoelectric activity signal to a zero mean value, determining the absolute value of the raw calibration myoelectric activity signal set to zero mean value, filtering the signal in absolute value to obtain the envelope therefrom, and from the envelope of the filtered signal, determining the minimum value and the maximum value of the envelope of the filtered signal.

6. System for controlling a limb of a virtual avatar (24) by the myoelectric activities of a limb (8) of a subject (10), the system comprising:

- a device (4) for measuring myoelectric activity signals resulting from contractions of first and second antagonistic muscles of the limb (8) of the subject (10), suitable for acquiring first and second raw calibration myoelectric activity signals resulting from contractions of variable intensities of first and second antagonistic muscles of the limb (8) of the subject (10) respectively, during a given period, and first and second raw myoelectric activity signals resulting from contractions of the two antagonistic muscles of the limb (8) of the subject (10), during a given period,
- a processing device (14) for processing the myoelectric activities comprising at least one display device (23) configured for displaying the virtual avatar (24), the processing device (14) being configured for implementing the method for controlling a limb of a virtual avatar (24) by the myoelectric activities of a limb (8) of a subject (10) according to any of the preceding claims.

7. System according to claim 6, wherein the device (4) for measuring comprises a plurality of electrodes (6) and is in the form of a bracelet.

8. System according to claim 6 or 7, furthermore comprising a sensory feedback device (16).

9. System according to claim 8, wherein the sensory feedback device (16) comprises a plurality of vibrating elements (18).

10. System according to claim 8 or 9, wherein the processing device (14) is configured for activating the sensory feedback device (16) selectively and discretely according to predetermined ranges of values of a sensory parameter.

11. System according to any one of claims 6 to 10, wherein the device (4) for measuring signals of myoelectric activity comprises a remote communication device (12) configured for remotely communicating with the processing device (14).

FIG.1

FIG.2

FIG.3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2014198034 A

**Littérature non-brevet citée dans la description**

- **AARON TABOR et al.** Designing Game-Based Myoelectric Prosthesis Training. *Design and Cognitive Impairment*, 2017

- **GAURAVKUMAR K PATEL et al.** Context-dependent adaptation improves robustness of myoelectric control for upper-limb prostheses. *Journal of Neural Engineering*, 2017, vol. 14 (5)